# Europäisches Patentamt

# European Patent Office

# Office européen des brevets

(11) Veröffentlichungsnummer: **0 170 965**
**B1**

(12) **EUROPÄISCHE PATENTSCHRIFT**

(45) Veröffentlichungstag der Patentschrift:
23.08.89

(21) Anmeldenummer: 85109147.0

(22) Anmeldetag: 22.07.85

(51) Int. Cl.⁴: **C 07 C 51/12**, C 07 C 51/54,
C 07 C 67/37, C 07 C 53/08,
C 07 C 53/12, C 07 C 69/14

(54) Verfahren zur gemeinsamen Herstellung von Carbonsäuren und Carbonsäureanhydriden sowie gegebenenfalls Carbonsäureestern.

(30) Priorität: 08.08.84 DE 3429179

(43) Veröffentlichungstag der Anmeldung:
12.02.86 Patentblatt 86/7

(45) Bekanntmachung des Hinweises auf die Patenterteilung:
23.08.89 Patentblatt 89/34

(84) Benannte Vertragsstaaten:
AT BE CH DE FR GB IT LI NL

(56) Entgegenhaltungen:
EP-A-0 026 280
EP-A-0 079 461

(73) Patentinhaber: HOECHST AKTIENGESELLSCHAFT,
Postfach 80 03 20, D-6230 Frankfurt am Main 80
(DE)

(72) Erfinder: Erpenbach, Heinz, Dr., Oberbuschweg 22,
D-5000 Köln 50 (DE)
Erfinder: Gehrmann, Klaus, Dr., Geschwister-
Scholl- Strasse 32, D-5042 Erftstadt (DE)
Erfinder: Hörstermann, Peter, Dr., Theodor- Heuss-
Strasse 2, D-5042 Erftstadt (DE)

**Beschreibung**

Die Erfindung betrifft ein Verfahren zur gemeinsamen Herstellung von Carbonsäuren und Carbonsäureanhydriden sowie ggf. Carbonsäureestern, insbesondere Essigsäure und Essigsäureanhydrid sowie ggf. Essigsäuremethylester.

Essigsäure und Essigsäureanhydrid gehören zu den wichtigsten aliphatischen Zwischenprodukten. Die überwiegende Menge wird zur Herstellung von Vinylacetat bzw. Celluloseacetat verwendet.

Essigsäure wurde früher fast ausschließlich durch Oxidation von Acetaldehyd, Alkanen oder Alkenen hergestellt. In jüngerer Zeit gewinnen jedoch Verfahren zur Herstellung von Essigsäure durch Carbonylierung von Methanol zunehmend an Bedeutung. So beschreibt die DE-1 767 151-C3 ein Verfahren zur Herstellung von Carbonsäuren und/oder deren Estern durch katalytische Umwandlung von gesättigten aliphatischen Alkoholen mit 1 bis 20 Kohlenstoffatomen oder deren Halogen-, Ester- oder Etherderivaten oder von Phenol, wobei man die genannten Ausgangsstoffe mit Kohlenmonoxid bei einem CO-Partialdruck von 0,34 bis 207 bar entweder in der Flüssigphase bei 100 bis 240°C oder in der Gasphase bei 200 bis 400°C in Gegenwart einer Rhodiumverbindung und von Brom, Jod, oder einer Verbindung dieser Halogene reagieren läßt und, falls ein Halogen-, Ester- oder Etherderivat eines Alkohols eingesetzt wird, in Anwesenheit von Wasser arbeitet.

Essigsäureanhydrid wird bisher überwiegend entweder durch Umsetzung von Essigsäure mit Keten oder durch eine modifizierte Acetaldehyd-Oxidation hergestellt. Neuere Wege beschreibt die DE-OS-2 441 502, die dadurch gekennzeichnet sind, daß man einen Carboxylatester oder einen Kohlenwasserstoffether mit einem Acyljodid oder -bromid unter praktisch wasserfreien Bedingungen umsetzt. Das Acylhalogenid wird durch Carbonylierung eines Alkylhalogenids in Gegenwart eines Katalysatorsystems aus einem Edelmetall der Gruppe VIII des Periodensystems und ggf. einem Promotor aus den Gruppen Ia, IIa, IIIa, IVb, VIb sowie Nichtedelmetallen der Gruppe VIII und Metallen der Lanthaniden und Actiniden des Periodensystems hergestellt.

Die DE-2 450 965-C2 beschreibt ein Verfahren zur Herstellung von Essigsäureanhydrid durch Umsetzen von Essigsäuremethylester oder Gemischen von Essigsäuremethylester und Dimethylether mit Kohlenmonoxid bei Drücken von 1 bis 500 bar und Temperaturen von 50 bis 250°C in Abwesenheit nennenswerter Wassermengen und in Gegenwart von Katalysatoren, die Edelmetalle der 8. Nebengruppe des Periodensystems oder deren Verbindungen, Jod und/oder Jodverbindungen sowie ggf. carbonylbildende Metalle enthalten, wobei man die Umsetzung in Gegenwart von Katalysatoren, die zusätzlich Alkyl- oder Arylphosphine und/oder organische Stickstoffverbindungen enthalten, und gegebenenfalls in Anwesenheit von 5 bis 50 Vol.-% Wasserstoff

durchführt. Der Einsatz von Methylacetat /Methanol-Gemischen, die 18 bis 20 % Methanol enthalten, wird besonders hervorgehoben.

Ein weiteres Verfahren zur gemeinsamen Herstellung von Essigsäure und Essigsäureanhydrid wird in der EP-0 087 869-A1 beschrieben. Dieses Verfahren ist dadurch gekennzeichnet, daß ein Carbonsäureester oder -ether, Wasser und ggf. ein Alkohol mit Kohlenmonoxid in Gegenwart eines Katalysators, bestehend aus einem Edelmetall der Gruppe VIII des Periodensystems, einem Brom- oder Jodpromotor und einem Copromotor aus einer Lewis-Base oder einem Nichtedelmetall, zu einem Gemisch aus Carbonsäure und Carbonsäureanhydrid umgesetzt werden, wobei der Wassergehalt der Einsatzmischung mindestens 5,5 Gew.-% beträgt. Die Gesamtmenge an Wasser und Alkohol soll jedoch 85 % der stöchiometrischen Menge an Ester und Ether nicht überschreiten.

Solche Reaktionsmischungen, die neben Essigsäure und reaktiven Jodverbindungen auch noch Wasser enthalten, sind jedoch hoch korrosiv gegenüber den meisten technischen Materialien, auch gegenüber Hastelloy-Edelstählen, so daß auf teurere Werkstoffe wie z. B. Tantal zurückgegriffen werden muß.

Dieser Nachteil kann durch den Einsatz eines wasserfreien Gemisches von Dimethylether und Methanol, das bei sämtlichen Methanol-Herstellern in ausreichender Menge zur Verfügung steht, vermieden werden. Es wurde nämlich überraschend gefunden, daß die Korrosivität der Reaktionsmischung wesentlich geringer ist, wenn unter wasserfreien Bedingungen gearbeitet werden kann. Die Aktivität des Katalysators bleibt gegenüber der wasserhaltigen Betriebsweise für die Gesamtheit der Produkte mindestens in gleicher Höhe erhalten bzw. ist sogar als verbessert anzusehen. Darüber hinaus liefert der Prozeß neben der Möglichkeit, das Produktverhältnis von Carbonsäure zu Carbonsäureanhydrid den wirtschaftlichen Erfordernissen anzupassen, als weiteres Handelsprodukt den entsprechenden Carbonsäureester.

Die vorliegende Erfindung betrifft daher ein Verfahren zur gemeinsamen Herstellung von Carbonsäuren und Carbonsäureanhydriden sowie ggf. Carbonsäureestern der allgemeinen Formeln RCOOH, RCOOCOR bzw. RCOOR, wobei R jeweils denselben Alkylrest mit 1 bis 4 C-Atomen bedeutet, welches dadurch gekennzeichnet ist, daß man Dialkylether der allgemeinen Formel ROR mit Alkoholen der allgemeinen Formel ROH im Molverhältnis 9 : 1 bis 1 : 9 unter wasserfreien Bedingungen mit Kohlenmonoxid und ggf. Wasserstoff in Gegenwart eines aus Carbonylkomplexen von Edelmetallen der Gruppe VIII des Periodensystems; einem Alkali-, Organophosphonium- oder Organoammoniumjodid; einem Alkyljodid der allgemeinen Formel RI; und ggf. aus Verbindungen carbonylbildender Nichtedelmetalle der Gruppen IV, V, VI, VII oder VIII des Periodensystems bestehenden Katalysatorsystems bei Temperaturen von 50 bis 250°C und Drücken von 0,1 bis 120 bar umsetzt.

Auf diese Weise kann man z. B. aus Dimethylether, Methanol und Methyljodid Essigsäure, Essigsäureanhydrid und ggf. Essigsäuremethylester herstellen. Das eingesetzte Kohlenmonoxid kann bis zu 10 Vol.-% Wasserstoff enthalten.

Ein besonderer Vorteil des Verfahrens liegt darin, daß sich durch Variation des Einsatzverhältnisses von Dialkylether zu Alkohol praktisch jedes Produktverhältnis von Carbonsäure zu Carbonsäureanhydrid einstellen läßt, so daß das Verfahren schnell an wechselnde Erfordernisse angepaßt werden kann.

Das zur Reaktion eingesetzte Kohlenmonoxid muß nicht unbedingt rein sein. Kleinere Mengen an Inertgasen wie Kohlendioxid, Stickstoff oder Methan stören die Carbonylierung nicht, wenn der Kohlenmonoxid-Partialdruck im Reaktor konstant gehalten wird. Ein Wasserstoffgehalt bis zu 10 % wirkt sich positiv auf die Katalysatoraktivität aus, verringert jedoch die Selektivität des Verfahrens durch Bildung von Hydrierungsprodukten wie z. B. Ethylidendiacetat oder Ethylenglykoldiacetat.

Als Katalysator kann jedes Edelmetall der Gruppe VIII des Periodensystems (Ru, Rh, Pd, Os, Ir, Pt) eingesetzt werden. Die höchste Aktivität besitzt jedoch das Rhodium. Als Einsatzform des Rhodiums bzw. der anderen Edelmetalle können alle Verbindungen eingesetzt werden, die unter den Reaktionsbedindungen löslich sind und den aktiven Edelmetallcarbonylkomplex bilden, z. B. $RhCl_3 \cdot 3H_2O$, $[Rh(CO)_2Cl]_2$, $[Pd(CO)_2I]_2$, $IrCl_3$, $Pd(CH_3CO_2)_2$, $PdCl_2$, $Pd(C_5H_7O_2)_2$. Die Konzentration der Edelmetallverbindung der Gruppe VIII in der Reaktionsmischung liegt vorzugsweise bei 0,001 bis 0,1 mol/l, insbesondere bei 0,005 bis 0,05 mol/l.

Von den als Promotorsalz eingesetzten Alkalijodiden kommt dem Lithiumjodid die größte Bedeutung zu, doch können auch NaI oder KI verwendet werden. Als Organophosphoniumjodid wird bevorzugt Methyltributylphosphoniumjodid eingesetzt, jedoch ist auch der Einsatz anderer quaternärer Phosphoniumjodide wie Methyltriphenylphosphoniumjodid, Tetrabutylphosphoniumjodid oder Dimethyldibutylphosphoniumjodid möglich. Als Organoammoniumverbindung wird bevorzugt das N,N-Dimethylimidazoliumjodid eingesetzt, jedoch sind auch N-Methylpyridiniumjodid, N-Methyl-3-picoliniumjodid, N-Methyl-2,4-lutidiniumjodid, N-Methyl-3,4-lutidiniumjodid, N-Methyl-chinoliniumjodid u.a. verwendbar. Die Konzentration des Promotorsalzes in der Reaktionsmischung kann zwischen 0,01 und 2 mol/l, vorteilhaft zwischen 0,1 und 0,8 mol/l liegen.

Die ggf. als Copromotoren verwendeten Carbonylkomplexe bildenden Nicht-Edelmetalle der Gruppen IV, V, VI, VII und VIII werden zweckmäßig in einer gut löslichen Form, z. B. als Acetylacetonat oder als Carbonyl in die Reaktion eingesetzt. Die Konzentrationen dieser Copromotoren in der Reaktionsmischung liegen zweckmäßig bei 0,01 bis 0,5 mol/l, bevorzugt bei 0,05 bis 0,3 mol/l. Es kommen hier vorzugsweise Verbindungen der Metalle Ti, Zr, V, Nb, Ta, Cr, Mo, W, Mn, Re, Fe, Co oder Ni infrage.

Das Alkyljodid RI, das wesentlicher Bestandteil des Katalysators ist, muß so ausgewählt werden, daß der Alkylrest R den Einsatzprodukten ROR (Ether) und ROH (Alkohol) entspricht, da sonst gemischte Produkte entstehen. Das bedeutet, daß zur Herstellung von Essigsäure, Methylacetat und Essigsäureanhydrid aus Dimethylether und Methanol Methyljodid eingesetzt werden muß, während bei der Herstellung von Propionsäure, Ethylpropionat und Propionsäureanhydrid aus Diethylether und Ethanol der Einsatz von Ethyljodid notwendig ist. Die Konzentration an Alkyljodid im Reaktionsgemisch kann bei 0,1 bis 5 mol/l, bevorzugt bei 0,5 bis 2,5 mol/l liegen.

Das Verfahren arbeitet bevorzugt in flüssiger Phase, kann jedoch auch in der Gasphase an auf Trägern gebundenen Katalysatoren angewendet werden. Die Reaktionstemperaturen liegen in beiden Fällen bevorzugt zwischen 150 und 250° C. Der Betriebsdruck liegt bevorzugt zwischen 20 und 60 bar. Das Carbonylierungsverfahren kann sowohl in einer diskontinuierlichen als auch in einer kontinuierlichen Anlage betrieben werden.

## Beispiel 1

In einem 1 Liter-Rührautoklav aus Edelstahl (Hastelloy B2) wurden 3 mol Dimethylether, gelöst in 3 mol Methanol, 1 mol Methyljodid, 0,2 mol Methyltributylphosphoniumjodid und 0,5 g Rhodium als $[Rh(CO)_2Cl]_2$ vorgelegt und durch Aufdrücken von Kohlenmonoxid ein Druck von 25 bar eingestellt. Nach dem Aufheizen auf die Reaktionstemperatur von 180°C wurde durch kontinuierliches Nachdrücken von Kohlenmonoxid 45 min lang ein Gesamtdruck von 50 bar aufrechterhalten. Nach dem Abkühlen und Entspannen ergab die gaschromatographische Analyse des Reaktionsgemisches einen Gehalt von 3 mol Essigsäure, 0,8 mol Methylacetat und 2,2 mol Essigsäureanhydrid.

## Beispiel 2

Beispiel 1 wurde wiederholt mit dem Unterschied, daß anstelle des Methyltributylphosphoniumjodids 0,2 mol N,N-Dimethylimidazoliumjodid als Promotor eingesetzt wurden. Dabei wurden im Produktgemisch 3 mol Essigsäure, 0,6 mol Methylacetat und 2,4 mol Essigsäureanhydrid nachgewiesen.

## Beispiel 3

Beispiel 1 wurde wiederholt mit dem Unterschied, daß anstelle des Methyltributylphosphoniumjodids 0,2 mol Lithiumjodid als Promotorsalz eingesetzt wurden. Das Produktgemisch enthielt 3 mol Essigsäure, 0,9 mol Methylacetat und 2,1 mol Essigsäureanhydrid.

## Beispiel 4

Beispiel 1 wurde unter Zusatz von 0,05 mol Zirkoniumacetylacetonat wiederholt. Nach Erreichen der Reaktionstemperatur von 180°C wurde durch kontinuierliches Aufdrücken von CO über 30 min ein Gesamtdruck von 50 bar eingestellt. Die Aufarbeitung des Produktgemisches ergab 3 mol Essigsäure, 0,4 mol Methylacetat und 2,6 mol Essigsäureanhydrid.

## Beispiel 5

Der Ansatz gemäß Beispiel 1 wurde unter Zusatz von 0,05 mol Vanadiumhexacarbonyl wiederholt und weiter gemäß Beispiel 4 behandelt. Die Aufarbeitung des Produktgemisches ergab 3 mol Essigsäure, 0,5 mol Methylacetat und 2,5 mol Essigsäureanhydrid.

## Beispiel 6

Der Ansatz gemäß Beispiel 1 wurde unter Zusatz von 0,05 mol Chromacetylacetonat wiederholt und weiter wie in Beispiel 4 behandelt. Nach der Aufarbeitung des Produktes wurden 3 mol Essigsäure, 0,3 mol Methylacetat und 2,7 mol Essigsäureanhydrid erhalten.

## Beispiel 7

Der Ansatz gemäß Beispiel 1 wurde unter Zusatz von 0,05 mol Dirheniumdecacarbonyl wiederholt und weiter wie in Beispiel 4 behandelt. Das Produktgemisch enthielt 3 mol Essigsäure, 0,4 mol Methylacetat und 2,6 mol Essigsäureanhydrid.

## Beispiel 8

Der Ansatz gemäß Beispiel 1 wurde unter Zusatz von 0,05 mol Dicobaltoctacarbonyl wiederholt und weiter wie in Beispiel 4 behandelt. Das Produktgemisch enthielt 3 mol Essigsäure, 0,5 mol Methylacetat und 2,5 mol Essigsäureanhydrid.

## Beispiel 9

In einem 1 Liter-Rührautoklav aus Edelstahl (Hastelloy B2) wurden 2 mol Dimethylether, gelöst in 4 mol Methanol, 1,2 mol Methyljodid, 0,2 mol Methyltributylphosphoniumjodid und 0,5 g Rhodium als $[Rh(CO)_2Cl]_2$ vorgelegt und dann 25 bar Kohlenmonoxid und 2,5 bar Wasserstoff aufgedrückt. Nach dem Aufheizen auf die Reaktionstemperatur von 180°C wurde durch kontinuierliches Nachdrücken eines Gasgemisches aus 90 Mol.-% Kohlenmonoxid und 10 Mol-% Wasserstoff 45 min lang ein Gesamtdruck von 50 bar aufrechterhalten.

Nach dem Abkühlen und Entspannen ergab sich aus der gaschromatographischen Analyse des Reaktionsgemisches ein Gehalt von 4,4 mol Essigsäure, 0,2 mol Methylacetat, 1,0 mol Essigsäureanhydrid und 0,4 mol Ethylidendiacetat.

## Beispiel 10

Beispiel 9 wurde wiederholt mit dem Unterschied, daß als Katalysator anstelle des Rhodiums 0,5 g Palladium als Palladiumacetylacetonat eingesetzt wurde. Das Produktgemisch enthielt dann 4,7 mol Essigsäure, 0,1 mol Methylacetat, 0,5 mol Essigsäureanhydrid und 0,7 mol Ethylidendiacetat.

## Beispiel 11

In dem beschriebenen Rührautoklav wurden 3 mol Diethylether, 2 mol Ethanol, 1 mol Ethyljodid, 0,2 mol Methyltributylphosphoniumjodid und 0,5 g Rhodium als $[Rh(CO)_2Cl]_2$ vorgelegt und durch Aufdrücken von Kohlenmonoxid ein Druck von 25 bar eingestellt. Nach dem Aufheizen auf 180°C wurde über einen Zeitraum von 3 h durch kontinuierliches Nachdrücken von Kohlenmonoxid der Reaktionsdruck bei 50 bar gehalten. Nach der Aufarbeitung wurden im Reaktionsprodukt 2 mol Propionsäure, 1,2 mol Ethylpropionat und 1,8 mol Propionsäureanhydrid nachgewiesen.

## Beispiel 12

In dem beschriebenen Rührautoklav wurden 3 mol Dimethylether, gelöst in 3 mol Methanol, 1 mol Methyljodid, 0,2 mol Tetrabutylphosphoniumjodid und 0,5 g Rhodium als $[Rh(CO)_2Cl]_2$ vorgelegt und durch Aufdrücken von Kohlenmonoxid ein Druck von 25 bar eingestellt. Nach dem Aufheizen auf die Reaktionstemperatur von 180°C wurde durch kontinuierliches Nachdrücken von Kohlenmonoxid 40 min lang ein Gesamtdruck von 50 bar aufrechterhalten. Nach dem Abkühlen und Entspannen ergab die gaschromatographische Analyse des Reaktionsgemisches einen Gehalt von 3 mol Essigsäure, 0,7 mol Methylacetat und 2,3 mol Essigsäureanhydrid.

**Beispiel 13**

5 mol Dimethylether, 1 mol Methanol, 1 mol Methyljodid, 0,2 mol Methyltributylphosphoniumjodid und 0,5 g Rhodium als [Rh(CO)$_2$Cl]$_2$ wurden eingesetzt. Nach Aufdrücken von 25 bar CO wurde die Reaktionsmischung auf 180°C aufgeheizt. Durch kontinuierliches Nachdrücken von CO wurde über 45 min lang ein Gesamtdruck von 50 bar aufrechterhalten. Nach Abkühlen und Entspannen ergab die gaschromatographische Analyse des Reaktionsgemisches einen Gehalt von 1 mol Essigsäure, 1,4 mol Methylacetat und 3,6 mol Essigsäureanhydrid.

**Beispiel 14**

1 mol Dimethylether, 5 mol Methanol, 1 mol Methyljodid, 0,2 mol Methyltributylphosphoniumjodid und 0,5 g Rhodium als [Rh(CO)$_2$Cl] wurden vorgelegt und wie in Beispiel 13 gearbeitet. Im erhaltenen Reaktionsprodukt wurden durch gaschromatographische Analyse 5 mol Essigsäure, 0,8 mol Methylacetat und 0,2 mol Essigsäureanhydrid nachgewiesen.

**Patentansprüche**

1. Verfahren zur gemeinsamen Herstellung von Carbonsäuren und Carbonsäureanhydriden sowie gegebenenfalls Carbonsäureestern der allgemeinen Formeln RCOOH, RCOOCOR bzw. RCOOR durch Umsetzung eines Dialkylethers der allgemeinen Formel ROR und eines Alkohols der allgemeinen Formel ROH mit Kohlenmonoxid, das gegebenenfalls bis zu 10 Vol.-% Wasserstoff enthält, in Gegenwart eines aus Carbonylkomplexen von Edelmetallen der Gruppe VIII des Periodensystems; einem Alkali-, Organophosphonium- oder Organoammoniumjodid; einem Alkyljodid der allgemeinen Formel RI, und gegebenenfalls aus Verbindungen carbonylbildender Nichtedelmetalle der Gruppen IV, V, VI, VII oder VIII des Periodensystems bestehenden Katalysatorsystems, wobei R jeweils denselben Alkylrest mit 1 bis 4 C-Atomen bedeutet, <u>dadurch gekennzeichnet</u>, daß man den Dialkylether und den Alkohol im Molverhältnis 9 : 1 bis 1 : 9 einsetzt und die Umsetzung unter wasserfreien Bedingungen bei Temperaturen von 50 bis 250°C und Drücken von 0,1 bis 120 bar durchführt.

2. Verfahren nach Anspruch 1, <u>dadurch gekennzeichnet</u>, daß man aus Dimethylether, Methanol und Methyljodid Essigsäure, Essigsäuremethylester und Essigsäureanhydrid herstellt.

**Claims**

1. A process for the common preparation of carboxylic acids and carboxylic anhydrides and, if appropriate, carboxylic acid esters of the general formulae RCOOH, RCOOCOR and RCOOR respectively, by reacting a dialkyl ether of the general formula ROR and an alcohol of the general formula ROH with carbon monoxide, optionally containing up to 10 % by volume of hydrogen, in the presence of a catalyst system comprising carbonyl complexes of noble metals of group VIII of the Periodic Table; an alkali metal iodide, organophosphonium iodide or organoammonium iodide; an alkyl iodide of the general formula RI and, if appropriate, compounds of carbonyl forming non-noble metals of groups IV, V, VI, VII or VIII of the Periodic Table, where R in each case denotes the same alkyl radical having 1 to 4 carbon atoms, which comprises employing the dialkyl ether and the alcohol in the molar ratio 9 : 1 to 1 : 9 and carrying out the reaction under anhydrous conditions at temperatures of from 50 to 250°C and pressures of from 0.1 to 120 bar.

2. The process as claimed in claim 1, wherein acetic acid, methyl acetate and acetic anhydride are prepared from dimethyl ether, methanol and methyl iodide.

**Revendications**

1. Procédé pour la fabrication simultanée d'acides carboxyliques et d'anhydrides carboxyliques ainsi éventuellement que d'esters d'acides carboxyliques de formules générales RCOOH, RCOOCOR ou RCOOR par réaction d'un oxyde de dialkyle de formule générale ROR et d'un alcool de formule générale ROH avec le monoxyde de carbone, qui contient éventuellement jusqu'à 10 % en volume d'hydrogène, en présence d'un système catalytique consistant en complexes carbonyles de métaux nobles du groupe VIII de la classification périodique; un iodure de métal acalin, d'organophosphonium ou d'organoammonium; un iodure d'alkyle de formule générale RI, et éventuellement un composé de métaux non nobles formant des carbonyles, des groupes IV, V, VI, VII ou VIII de la classification périodique, dans lequel R représente chaque fois le même reste alkyle en C$_1$-C$_4$, caractérisé en ce que l'on utilise l'oxyde de dialkyle et l'alcool dans un rapport molaire de 9 : 1 à 1 : 9 et on met en oeuvre la réaction en conditions anhydres à des températures de 50 à 250°C et sous des pressions de 0,1 à 120 bar.

2. Procédé selon la revendication 1, caractérisé en ce que l'on fabrique l'acide acétique, l'acétate de méthyle et l'anhydride acétique à partir d'oxyde de diméthyle, de méthanol et d'iodure de méthyle.